# EUROPEAN PATENT SPECIFICATION

(11) **EP 1 673 388 B1**
(45) Date of publication and mention of the grant of the patent: **26.11.2008**
(21) Application number: 04791294.4
(22) Date of filing: 22.10.2004
(51) Int. Cl.: C07K 14/54, C12N 15/24, C12N 15/62, A61K 38/20, A61K 31/713

(54) **CXCL8 ANTAGONISTS**
CXCL8-ANTAGONISTEN
ANTAGONISTES DE LA CXCL8

(30) Priority: 22.10.2003 EP 03103909
(43) Date of publication of application: 28.06.2006
(73) Proprietor: Laboratoires Serono SA, 1267 Coinsins, Vaud (CH)
(72) Inventor: PROUDFOOT, Amanda, F-74140 Chens Sur Leman (FR); SHAW, Jeffrey, CH-1234 Vessy (CH)
(74) Representative: von Ballmoos, Prisca
(86) International application number: PCT/EP2004/052637
(87) International publication number: WO 2005/040210

(56) References cited:
- WO-A-02/28419
- WO-A-93/11159
- WO-A-96/09062
- LORTAT-JACOB HUGUES ET AL: "Structural diversity of heparan sulfate binding domains in chemokines." PROCEEDINGS OF THE NATIONAL ACADEMY OF SCIENCES OF THE UNITED STATES OF AMERICA. UNITED STATES 5 FEB 2002, vol. 99, no. 3, 5 February 2002 (2002-02-05), pages 1229-1234, XP002273719 ISSN: 0027-8424 cited in the application
- JOHNSON Z ET AL: "A novel strategy for blocking chemokine mediated inflammation" INFLAMMATION RESEARCH 01 JUL 2003 SWITZERLAND, vol. 52, no. SUPPL., 1 July 2003 (2003-07-01), pages S187-S189, XP008028536 ISSN: 1023-3830
- KUSCHERT G S ET AL: "Identification of a glycosaminoglycan binding surface on human interleukin-8." BIOCHEMISTRY. UNITED STATES 11 AUG 1998, vol. 37, no. 32, 11 August 1998 (1998-08-11), pages 11193-11201, XP002273720 ISSN: 0006-2960 cited in the application
- FREVERT CHARLES W ET AL: "Binding of interleukin-8 to heparan sulfate and chondroitin sulfate in lung tissue." AMERICAN JOURNAL OF RESPIRATORY CELL AND MOLECULAR BIOLOGY. UNITED STATES APR 2003, vol. 28, no. 4, April 2003 (2003-04), pages 464-472, XP008028514 ISSN: 1044-1549 cited in the application
- PROUDFOOT A E I ET AL: "STRATEGIES FOR CHEMOKINE ANTAGONISTS AS THERAPEUTICS" SEMINARS IN IMMUNOLOGY, W.B. SAUNDERS COMPANY, PA, US, vol. 15, no. 1, 2003, pages 57-65, XP001156225 ISSN: 1044-5323

## Description

### FIELD OF THE INVENTION

The invention relates to structure and the properties of novel antagonists of the chemokine CXCL8.

### BACKGROUND OF THE INVENTION

Chemokines are small, secreted pro-inflammatory proteins, which mediate directional migration of leukocytes from the blood to the site of injury. Depending on the position of the conserved cysteines characterizing this family of proteins, the chemokine family can be divided structurally into C, C-C, C-X-C and C-X₃-C chemokines which bind to a series of membrane receptors ( Baggiolini M et al., 1997; Fernandez EJ and Lolis E, 2002).

These membrane proteins, all heptahelical G-protein coupled receptors, allow chemokines to exert their biological activity on the target cells, which may present specific combinations of receptors according to their state and/or type. The physiological effects of chemokines result from a complex and integrated system of concurrent interactions: the receptors often have overlapping I igand specificity, so that a single receptor can bind different chemokines, as well a single chemokine can bind to different receptors.

Usually chemokines are produced at the site of injury and cause leukocyte migration and activation, playing a fundamental role in inflammatory, immune, homeostatic, hematopoietic, and angiogenic processes. Thus, these molecules are considered good target candidates for therapeutic intervention in diseases associated with such processes. The inhibition of chemokines, or of their receptors, can reduce excessive leukocyte maturation, recruitment and activation, as well as other pathological degenerations related to angiogenesis or arteriosclerosis (Baggiolini M, 2001; Loetscher P and Clark-Lewis I, 2001; Godessart N and Kunkel SL, 2001).

Studies on structure-activity relationships indicate that chemokines have two main sites of interaction with their receptors, the flexible amino-terminal region and the conformationally rigid loop that follows the second cysteine. Chemokines are thought to dock onto receptors by means of the loop region, and this contact is believed to facilitate the binding of the amino-terminal region that results in receptor activation. This importance of the amino-terminal region has been also demonstrated by testing natural and synthetic chemokines in which this domain is modified or shortened. This processing, following proteolytic digestion, mutagenesis, or chemical modification of amino acids, can either activate or render these molecules inactive, generating compounds with agonistic and/or antagonistic activity. Thus, chemokines with specific modifications in the amino-terminal region have therapeutic potential for inflammatory and autoimmune diseases (Schwarz and Wells, 1999).

Chemokines, like other cell-signaling soluble molecules (interleukins, growth factors), have physiologically important interactions not only with cell receptors but also with glycosaminoglycans (GAGs), although with varying affinities. These negatively charged molecules are formed by disaccharide repeats (such as heparin, chondroitin sulfate, heparan sulfate, dermatan sulfate, and hyaluronic acid) and naturally occur on cell surfaces, in the extracellular matrix, or in the circulation. They can be present in isolated forms or linked to proteins (Proteoglycans, or PGs) following the posttranslational addition of GAGs at serine residues.

Chemokines have basic residues (mainly Arginine and Lysine) clustered in short portions of their sequence which are suitable for this purpose but such motifs are structured in different manner for each chemokine, or group of highly homologous chemokines. Some of these GAG-binding sites have been associated to specific consensus sequences, such as BBXB motifs (where B represen ts a basic residue, and X any other residue) or other arrangements (Kuschert G et al., 1999; Proudfoot A et al., 2001; Proudfoot A et al., 2003).

The main consequence of GAGs-chemokine interaction seems to be the aggregation of the chemokines, a state that can provide a protection from proteolysis, but it can also modulate the gradient-generating release of the chemokines in the circulation and consequently their presentation to the receptors (Hoogewerf AJ et al., 1997; Kuschert G et al., 1999). The interaction with GAGs and the formation of these gradients has been clearly demonstrated for many chemokines, and the relative affinity has been measured. Therefore, it has been suggested that also the modulation of such interactions may represent a therapeutic approach in inflammatory disease (Ali S et al., 2001; Patel D et al., 2001).

Means to achieve a therapeutic effect on the basis of the GAGs-chemokines interactions known in the art involve the generation of GAGs analogs that modulate the interaction between endogenous GAGs and chemokines (WO 94/20512), the use of heparanase for eliminating GAGs (WO 97/11684), the administration of chemokine-GAGs complexes (WO 99/62535), the modification of GAGs binding domain with polymers (WO 02/04015), or the substitution of residues involved in GAG-binding activity (WO 02/28419, WO 03/051921).

Even though extensive studies have been performed on some chemokines, it is well established that is not possible to anticipate, on the basis of the sequence homology with chemokine having limited similarity or known GAGs-binding protein motifs, which specific basic residues have to be modified with non -conservative substitutions to impair GAG-binding, since there is a significant structural diversity of GAG-binding domains amongst the chemokine protein family (Lortat-Jacob H et al., 2002).

Amongst chemokines, CXCL8 (also known as Interleukin-8, IL-8, monocyte-derived neutrophil chemotactic factor, MDNCF, Neutrophil-Activating Protein 1, NAP-1, lymphocyte-derived neutrophil-activating factor, LYNAP, neutrophil-activating factor, NAF, granulocyte chemotactic protein 1, GCP-1, Emoctakin) is known as a potent chemotactic inflammation-mediating factor exerting its activity not only on neutrophils, but also on lymphocytes, monocytes, endothelial cells, and fibroblasts (Mukaida N, 2003; Shi Q et al., 2001; Zeilhofer HU and Schorr W, 2000; Atta-ur-Rahman H and Siddiqui RA, 1999).

CXCL8 is produced from various types of cells in response to a wide variety of inflammatory stimuli: cytokines, microbial products, environmental changes (such as hypoxia, acidosis, hyperglycemia, hyperosmotic pressure, high cell density, hyperthermia, radiation, chemotherapeutic agents, or reperfusion). It has also been shown that CXCL8 is motogenic, mitogenic, and angiogenic, suggesting that CXCL8 plays an important role in human tumor progression.

By activating its receptors (CXCR2 and CXCR1), CXCL8 mediates several intracellular events associated to numerous pathophysiological processes, such as in host defense mechanism. CXCL8 activates also signal transduction processes leading to desensitization, internalization, and recycling of CXCR2/CXCR1.

The discovery of these biological functions suggests that CXCL8 is an important mediator of various pathological conditions such as chronic inflammation and cancer, implying that blockade of its actions could be exploited for therapeutic purposes. The literature provides many examples of molecules inhibiting CXCL8, including CXCL8 - derived mutants or peptides (WO 91/08231, WO 93/11159, WO 96/09062; Moser B et al., 1993).

CXCL8-GAGs interactions have been studied, also by the means of CXCL8 mutants which are truncated or have single amino acid substitutions, to characterize their involvement in CXCL8 properties, such as the retention in specific tissues (Frevert C et al., 2003; Frevert C et al., 2002) and receptor / heparin binding (Goger B et al., 2002; Spillmann D et al., 1998; Kuschert GS, et al., 1998; Kuschert GS, et al., 1997; Hoogewerf AJ et al., 1997; Skelton N et al., 1999; Dias-Baruffi M et al., 1998; Witt D and Lander A, 1994; Webb L et al., 1993), identifying GAG-binding motifs in the 20's loop and C-terminal regions of CXCL8. However, none of these approaches identified CXCL8 variants having the *in vivo* antagonistic effects against CXCL8.

### SUMMARY OF THE INVENTION

It has been surprisingly found that specific combinations of basic residues in the carboxyl-terminus of human CXCL8 polypeptide can be substituted to generate CXCL8 antagonists. The elimination of these basic residues by non-conservative substitutions (for example, with Alanines) leads to the generation of CXCL8 mutant sequences having antagonistic activities against CXCL8 *in vivo*. Compounds prepared in accordance with the present invention can be used to block the activity of CXCL8 on CXCL8-binding cells, thereby providing the rapeutic compositions for use in the treatment of CXCL8-related diseases, in particular for autoimmune, inflammatory, or infectious diseases.

The invention further provides the nucleic acid encoding the CXCL8 mutant sequences, together with the vectors and host cells for expressing them and the process for their preparation.

The CXCL8 antagonists of the Invention can be provided in various alternative forms, such as the active mutants, fusion proteins comprising an amino acid sequence belonging to a prote in other than CXCL8, as well as the corresponding molecules in the form of their encoding nucleic acids, host cells expressing them, active fractions, precursors, salts, derivatives, complexes or conjugates.

The polypeptides comprising the CXCL8 mutant sequences described herein can be provided in purified preparations and are useful as a medicament. In particular, the polypeptides comprising CXCL8-1B3 (SEQ ID NO: 4), or CXCL8-2B3 (SEQ ID NO: 6) are useful as medicaments.

The invention further includes pharmaceutcal compositions containing a CXCL8 antagonist described herein as active ingredient, and their use for preparing the compositions that are useful for the treatment of CXCL8-related diseases, in particular autoimmune, inflammatory or infectious diseases.

The CXCL8 antagonists described herein are further useful in methods for treatment of autoimmune, inflammatory and infectious diseases. Such methods comprise the administration of an effective amount of a CXCL8 antagonist of the invention.

Other features and advantages of the invention will be apparent from the following detailed description.

### DESCRIPTION OF THE FIGURES

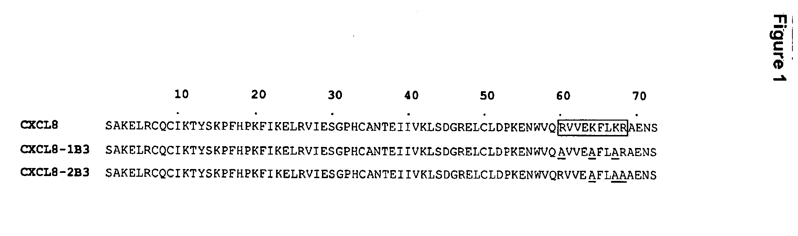

- Figure 1:: amino acid sequences of mature human CXCL8 (CXCL8; SEQ ID NO: 2), and of the mutants generated on the basis of these sequence, CXCL8-1B3 (SEQ ID NO: 4) and CXCL8-2B3 (SEQ ID NO: 6), which have been expressed and tested as described in the Examples (mutated amino acids are underlined; the numbering is based on the mature human sequence). The cluster of basic amino acids in CXCL8 sequence is boxed.

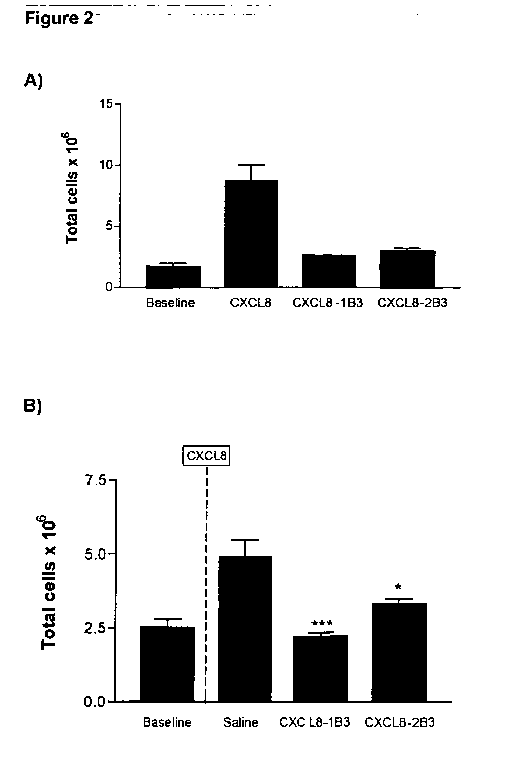

- Figure 2:: A) Graph-summarizing-the effects of CXCL8 and of CXCL8 mutants in the *in vivo* peritoneal cell recruitment model, compared with baseline, at 4 hours (the data are expressed as mean total cells ± standard error; n=3 mice per group). B) Graph summarizing the inhibiting effects of CXCL8 mutants o n CXCL8-induced cell recruitment in the peritoneal cavity, compared to saline treatment (the data are expressed as mean total cells ± standard error; n = 3 mice per group. *p* < 0.05 *, *p* < 0.001 ***).

### DETAILED DESCRIPTION OF THE INVENTION

The main object of the present invention is to provide novel antagonists of CXCL8 comprising a mutant sequence of human mature CXCL8 polypeptide, characterized in that the three basic residues Arginine 60, Lysine 64 and Lysine 67 of said polypeptide are substituted to Alanine, Glycine, Serine, Threonine, Proline, Glutammic Acic, Glutamine, Aspartic Acid, or Asparagine. Another basic residue that can be additionally mutated in the same way in preferred mutants shown in the examples is Arginine 68),.

More in particular, recombinant CXCL8 mutant sequencers, having specific combinations of basic residues substituted with Alanines are active as CXCL8 antagonists. These mutant has the sequence of CXCL8-1B3 (SEQ ID NO: 4), wherein Arginine60-Lysine64-Lysine67 are mutated to Alanine. Also disclosed is a mutant having the sequence of CXCL8-2B3 (SEQ ID NO:6).

CXCL8 mutants prepared in accordance with the present invention can be used to block the activity of CXCL8*in vivo*, thereby providing therapeutic compositions for use in the treatment of CXCL8-related diseases, due to excessive or uncontrolled CXCL8 production, in particular autoimmune, infectious, or inflammatory diseases.

The amino acid replacing the specific combinations of basic residue is preferably a non-polar, small amino acid like Alani ne or Glycine, but other amino acids are appropriate, provided that they have a charge and dimension which are incompatible with GAG-binding and, at the same time, poorly interfere with other properties of the protein. Amino acids suitable for the substitutions are Serine, Threonine, Proline, Glutammic Acic, Glutamine, Aspartic acid, or Asparagine.

Further objects of the present invention are alternative active molecules that can be generated on the basis of the CXCL8 antagonists disclosed above following the technical teachings in the prior art, and that can be used as active ingredients in pharmaceutical compositions.

The term "active" means that such alternative compounds should maintain , or even potentiate, the antagonistic properties of the CXCL8 mutant sequences of the invention, i.e. it should antagonize CXCL8 *in vivo* activities such as peritoneal cell recruitment.

The properties of the CXCL8 antagonists defined above, and exemplified herein using CXCL8-1B3 or CXCL8-2B3 as CXCL8 antagonist, can be maintained, or even potentiated, in the active mutants. This category of molecules includes natural or synthetic analogs of said sequence, wherein one or more amino acid residues have been added, deleted, or substituted, provided they display the same biolo gical activity characterized in the present invention at comparable or higher levels, as determined by means known in the art and disclosed in the Examples below.

Natural analogs are intended, for example, CXCL8 sequences in other organisms, like mouse, mutated in the position indicated by the Invention. Artificial analogs are intended peptides and polypeptides generated by site-directed mutagenesis techniques, combinatorial technologies at the level of encoding DNA sequence (such as DNA shuffling, phage display/selection), or by computer-aided design studies, or any other known technique suitable thereof, which provide a finite set of substantially corresponding mutated or shortened peptides or polypeptides. These alternative molecules can be routinely obtained and tested by one of ordinary skill in the art using the teachings presented in the prior art and in the Examples below.

For example, specific artificial mutants may have one or more amino acids being added, deleted, or substituted in the amino-terminal region known to affect receptor binding. In particular, these mutations may involve one or more of the first six amino acids of the mature human CXCL8 positioned in the amino-terminal region, just before the conserved CXC motif (fig. 1).

In accordance with the present invention, preferred changes in these active mutants are commonly known as "conservative" or "safe" substitutions, and involve non-basic residues. Conservative amino acid substitutions are those with amino acids having sufficiently similar chemical properties, in order to preserve the structure and the biological function of the molecule. It is clear that insertions and deletions of amino acids may also be made in the above defined sequences without altering their function, particularly if the insertions or deletions only involve a few amino acids, e.g., under ten, and preferably under three, and do not remove or displace amino acids which are critical to the functional conformation of a protein or a peptide.

The literature provide many models on which the selection of conservative amino acids substitutions can be performed on the basis of statistical and physico -chemical studies on the sequence and/or the structure of natural protein (Rogov SI and Nekrasov AN, 2001). Protein design experimen ts have shown that the use of specific subsets of amino acids can produce foldable and active proteins, helping in the classification of amino acid "synonymous" substitutions which can be more easily accommodated in protein structure (Murphy LR et al., 200). The synonymous amino acid groups and more preferred synonymous groups for the substitutions are those defined in Table I.

Specific variants of the CXCL8 antagonists of the invention can be obtained in the form of peptide mimetics (also called peptidomi metics) of the disclosed mutants of CXCL8, in which the nature of peptide or polypeptide has been chemically modified at the level of amino acid side chains, of amino acid chirality, and/or of the peptide backbone. These alterations are intended to provide antagonists with improved preparation, potency and/or pharmacokinetics features.

For example, when the peptide is susceptible to cleavage by peptidases following injection into the subject is a problem, replacement of a particularly sensitive peptide bond with a non-cleavable peptide mimetic can provide a peptide more stable and thus more useful as a therapeutic. Similarly, the replacement of an L -amino acid residue is a standard way of rendering the peptide less sensitive to proteolysis, and finally more similar to organic compounds other than peptides. Also useful are amino-terminal blocking groups such as t-butyloxycarbonyl, acetyl, theyl, succinyl, methoxysuccinyl, suberyl, adipyl, azelayl, dansyl, benzyloxycarbonyl, fluorenylmethoxycarbonyl, methoxyazelayl, methoxyadipyl, methoxysuberyl, and 2,4-dinitrophenyl. Many other modifications providing increased potency, prolonged activity, easiness of purification, and/or increased half-life are known in the art (WO 02/10195: Villain M et al., 2001).

Preferred alternative, "synonymous" groups for amino acids derivatives included in peptide mimetics are those defined in Table II. A non-exhaustive list of amino acid derivatives also include aminoisobutyric acid (Aib), hydroxyproline (Hyp), 1,2,3,4-tetrahydro-isoquinoline-3-COOH, indoline-2carboxylic acid, 4-difluoro-proline, L-thiazolidine-4-carboxylic acid, L-homoproline, 3,4-dehydro-proline, 3,4-dihydroxy-phenylalanine, cyclohexyl-glycine, and phenylglycine.

By "amino acid derivative" is intended an amino acid or amino acid-like chemical entity other than one of the 20 genetically encoded naturally occurring amino acids. In particular, the amino acid derivative may contain substituted or non-substituted alkyl moieties that can be linear, branched, or cyclic, and may include one or more heteroatoms. The amino acid derivatives can be made de novo or obtained from commercial sources (Calbiochem-Novabiochem AG, Switzerland; Bachem, USA).

The techniques for the synthesis and the development of peptide mimetics, as well as non-peptide mimetics, are well known in the art (Hruby VJ and Balse PM, 2000; Golebiowski A et al., 2001). Various methodology for incorporating unnatural amino acids into proteins, using both in vitro and in vivo translation systems, to probe and/or improve protein structure and function are also disclosed in the literature (Dougherty DA, 2000).

Still specific variants of the CXCL8 antagonists of the invention are the ones comprising one of the amino acid sequence as defined above and an amino acid sequence belonging to a protein sequence other than the human mature CXCL8. This heterologous latter sequence should provide additional properties without impairing significatively the antagonistic activity, or improving GAGs-binding properties. Examples of such additional properties are an easier purification procedure, a longer lasting half-life in body fluids, an additional binding moiety, the maturation by means of an endoproteolytic digestion, or extracellular localization. This latter feature is of particular importance for defining a specific group of fusion or chimeric proteins included in the above definition since it allows the molecules defined as CXCL8 antagonists in this invention to be localized in the space where not only where the isolation and purification of these polypeptides is facilitated, but also where CXCL8 and -its receptors naturally interact.

Design of the moieties, ligands, and linkers, as well methods and strategies for the construction, purification, detection and use of fusion pr oteins are widely discussed in the literature (Nilsson J et al., 1997; "Applications of chimeric genes and hybrid proteins" Methods Enzymol. Vol. 326-328, Academic Press, 2000; WO 01/77137). Additional protein sequences which can be used to generate the antagonists of the present invention are chosen amongst extracellular domains of membrane -bound protein, immunoglobulin constant region (Fc region), multimerization domains, signal peptides, export signal-containing proteins, and tag sequences (e.g. histidin e tag). The choice of one or more of these sequences to be fused to the CXCL8 mutant of the invention is functional to specific use and/or purification protocol of said agent.

For example, fusion proteins comprising CXCL8-1B3 or CXCL8-2B3 can be obtained by linking this sequence to an immunoglobulin domain constant region, a protein domain known to improve the stability and the efficacy of recombinant proteins in the circulation. The resulting fusion protein can be expressed directly by mammalian cells (such as CHO or HEK293 cells) using the appropriate expression vectors so that the fusion protein is secreted in the culture medium. In a preferred arrangement, the nucleic acid sequence encoding the mature CXCL8-1B3 or CXCL8-2B3 can be cloned in an expression vector fused to a nucleic acid sequence encoding the human CXCL8 signal sequence (or any other appropriate signal sequence) at its 5' end, and the nucleic acid sequence encoding the constant region (segment 243-476) of human immunoglobulin lambda heavy chain IgG1 (NCBI Acc. No. CAA75302) at its 3' end. The resulting vector can be used to transform a CHO or HEK293 cell line and the clones stably expressing and secreting the recombinant fusion protein having CXCL8 - 1B3 or CXCL8-2B3 at the N-terminus and the IgG1 sequence at the C-terminus can be selected. This clone then can be used for scaling up the production and for purifying the recombinant fusion protein from the culture medium. Alternatively, the position of the nucleic acid encoding the constant region of human immunoglobulin lambda heavy chain IgG1 and CCL2-P8A can be inversed, and the resulting protein can be expressed and secreted using still the human CXCL8 signal sequence, or any other appropriate signal sequence.

The CXCL8 mutant sequences acting as CXCL8 antagonists of the present invention can be in other alternative forms which can be preferred according to the desired method of use and/or production, for example as active fractions, precursors, salts, derivatives, conjugates or complexes.

The term "fraction" refers to any fragment of the polypeptidic chain of the compound itself, alone or in combination with related molecules or residues bound to it, for example residues of sugars or phosphates. Such molecules can result also from other modifications which do not normally alter primary sequence, for example *in vivo* or *in vitro* chemical derivativization of peptides (acetylation or carboxylation), those made by modifying the pattern of phosphorylation (introduction of phosphotyrosine, phosphoserine, or phosphothreonine residues) or glycosylation (by exposing the peptide to enzymes which affect glycosylation e.g., mammalian glycosylating or deglycosylating enzymes) of a peptide during its synthesis and processing or in further processing steps.

The "precursors" are compounds which can be converted into the compounds of present invention by metabolic and enzymatic processing prior or after the administration to the cells or to the body.

The term "salts" herein refers to both salts of carboxyl groups and to acid addition salts of amino groups of the peptides, polypeptides, or analogs thereof, of the present invention. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid. Any of such salts should have substantially similar activity to the peptides and polypeptides of the invention or their analogs.

The term "derivatives" as herein used refers to derivatives which can be prepared from the functional groups present on the lateral chains of the amino acid moieties or on the amino-/ or carboxy-terminal groups according to known methods. Such derivatives include for example esters or aliphatic amides of the carboxyl -groups and N-acyl derivatives of free amino groups or O-acyl derivatives of free hydroxyl-groups and are formed with acyl-groups as for example alcanoyl- or aroyl-groups.

Useful conjugates or complexes of obligate monomeric variants of homodimer-forming chemokines defined above can be generated, using molecules and methods known in the art of the interaction with receptor or other proteins (radioactive or fluorescent labels, biotin), therapeutic efficacy (cytotoxic agents), or improving the agents in terms of drug delivery efficacy, such as polyethylene glycol and other natural or synthetic polymers (Harris JM and Chess RB, 2003; Greenwald RB et al., 2003; Pillai O and Panchagnula R, 2001). These alternative CXCL8 antagonists may be produced following a site-directed modification of an appropriate residue, in an internal or terminal position. Residues can be used for attachment, provided they have a side-chain amenable for polymer attachment (i.e., the side chain of an amino acid bearing a functional group, e.g., lysine, aspartic acid, glutamic acid, cysteine, histidine, etc.). Alternatively, a residue at these sites can be replaced with a different amino acid having a side chain amenable for polymer attachment (for example, a Cysteine for allowing PEGylation). Also, the side chains of the genetically encoded amino acids can be chemically modified for polymer attachment, or unnatural amino acids with appropriate side chain functional groups can be employed. Polymer attachment may be not only to the side chain of the amino acid naturally occurring in a specific position of the antagonist or to the side chain of a natural or unnatural amino acid that replaces the amino acid naturally occurri ng in a specific position of the antagonist, but also to a carbohydrate or other moiety that is attached to the side chain of the amino acid at the target position.

Polymers suitable for these purposes are biocompatible, namely, they are non - toxic to biological systems, and many such polymers are known. Such polymers may be hydrophobic or hydrophilic in nature, biodegradable, non -biodegradable, or a combination thereof. These polymers include natural polymers (such as collagen, gelatin, cellulose, hyaluronic acid), as well as synthetic polymers (such as polyesters, polyorthoesters, polyanhydrides). Examples of hydrophobic non-degradable polymers include polydimethyl siloxanes, polyurethanes, polytetrafluoroethylenes, polyethylenes, polyvinyl chlorides, and polymethyl methaerylates. Examples of hydrophilic non-degradable polymers include poly(2-hydroxyethyl methacrylate), polyvinyl alcohol, poly(N-vinyl pyrrolidone), polyalkylenes, polyacrylamide, and copolymers thereof. Preferred polymers comprise as a sequential repeat unit ethylene oxide, such as polyethylene glycol (PEG).

The preferred method of attachment employs a combination of peptide synthesis and chemical ligation. Advantageously, the attachment of a water-soluble polymer will be through a biodegradable linker, especially at-the amino-terminal region of a protein. Such modification acts to provide the protein in a precursor (or "pro-drug") form, that, upon degradation of the linker releases the protein without polymer modification.

The antagonists of the invention may be prepared by any procedure known in the art, including recombinant DNA-related technologies, and chemical synthesis technologies.

Another object of the invention are the nucleic acid molecules comprising the nucleic acid sequences coding for the antagonists of CXCL8 chemokines described above, including nucleotide sequences substantially the same, for example the coding sequence of the tested CXCL8 mutant (SEQ ID NO: 3). The coding sequence of another tested CXCL8 mutant (SEQ ID NO: 5) is also disclosed. These sequences encode also for the natural CXCL8 signal sequence that is eliminated before the secretion.

"Nucleotide sequences substantially the same" includes all other nucleic acid sequences that, by virtue of the degeneracy of the genetic code, also code for the given amino acid sequences.

Still another object of the invention are expression vectors which comprise the above nucleic acids, host cells transformed with such vectors, and the process of preparation of the antagonists described above, comprising culturing these transformed cells and collecting the expressed proteins. When the vector expresses the antagonists as a fusion protein with extracellular, export signal, or signal-peptide containing proteins, CXCL8 antagonists can be secreted in the extracellular space, and can be more easily collected and purified from cultured cells in view of further processing or, alternatively, the cells can be directly used or administered. The example describes such expression vectors as well as host cells and the process of preparation.

These other objects of the invention can be achieved by combining the disclosure provided herein with the knowledge of common molecular biology techniques. Many books and reviews provides teachings on how to clone and produce recombinant proteins using vectors and Prokaryotic or Eukaryotic host cells, such as some titles in the series "A Practical Approach" published by Oxford University Press ("DNA Cloning 2: Expression Systems", 1995; "DNA Cloning 4: Mammalian Systems", 1996; "Protein Expression", 1999; "Protein Purification Techniques", 2001).

The DNA sequence coding for the proteins of the invention can be inserted and ligated into a suitable episomal or non-/homologously integrating vectors, which can be introduced in the appropriate host cells by any suitable means (transformation, transfection, conjugation, protoplast fusion, electroporation, calcium phosphate-precipitation, direct microinjection, etc.) to transform them. Factors of importance in selecting a particular plasmid or viral vector include: the ease with which recipient cells that contain the vector, may be recognized and selected from those recipient cells which do not contain the vector; the number of copies of the vector which are desired in a particular host; and whether it is desirable to be able to "shuttle" the vector between host cells of different species.

The vectors should allow the expression of the isolated or fusion protein including the antagonist of the invention in the prokaryotic or eukaryotic host cell under the control of transcriptional initiation / termination regulatory sequences, which are chosen to be constitutively active or inducible in said cell. A cell line substantially enriched in such cells can be then isolated to provide a stable cell line.

For eukaryotic hosts (e.g. yeasts, insect or mammalian cells), different transcriptional and translational regulatory sequences may be employed, depending on the nature of the host. They-may be derived form viral sources, such as adenovirus, bovine papilloma virus, Simian virus or the like, where the regulatory signals are associated with a particular gene which has a high level of expression. Examples are the TK promoter of the Herpes virus, the SV40 early promoter, the yeast gal4 gene promoter, etc. Transcriptional initiation regulatory signals may be selected which allow for repression and activation, so that expression of the genes can be modulated. The cells which have been stably transformed by the introduced DNA can be selected by also introducing one or more markers which allow for selection of host cells which contain the expression vector. The marker may also provide for phototrophy to an auxotropic host, biocide resistance, e.g. antibiotics, or heavy metals such as copper, or the like. The selectable marker gene can either be directly linked to the DNA gene sequences to be expressed, or introduced into the same cell by co -transfection. Additional elements may also be needed for optimal synthesis of proteins of the invention.

Host cells may be either prokaryotic or eukaryotic. Preferred are eukaryotic hosts, e.g. mammalian cells, such as human, monkey, mouse, and Chinese Hamster Ovary (CHO) cells, because they provide post-translational modifications to protein molecules, including correct folding or glycosylation at correct sites. Also yea st cells can carry out post-translational peptide modifications including glycosylation. A number of recombinant DNA strategies exist which utilize strong promoter sequences and high copy number of plasmids that can be utilized for production of the desired d proteins in yeast. Yeast recognizes leader sequences in cloned mammalian gene products and secretes peptides bearing leader sequences (i.e., pre-peptides).

For long-term, high-yield production of a recombinant polypeptide, stable expression is preferred. For example, cell lines which stably express the polypeptide of interest may be transformed using expression vectors which may contain viral origins of replication and/or endogenous expression elements and a selectable marker gene on the same or on a sepa rate vector. Following the introduction of the vector, cells may be allowed to grow for 1-2 days in an enriched media before they are switched to selective media. The purpose of the selectable marker is to confer resistance to selection, and its presence allows growth and recovery of cells that successfully express the introduced sequences. Resistant clones of stably transformed cells may be proliferated using tissue culture techniques appropriate to the cell type. A cell line substantially enriched in such cells can be then isolated to provide a stable cell line.

Mammalian cell lines available as hosts for expression are known in the art and include many immortalised cell lines available from the American Type Culture Collection (ATCC) including, but not limited to, Chinese hamster ovary (CHO), HeLa, baby hamster kidney (BHK), monkey kidney (COS), C127, 3T3, BHK, HEK 293, Bowes melanoma and human hepatocellular carcinoma (for example Hep G2) cells and a number of other cell lines. In the baculovirus system, the materials for baculovirus/insect cell expression systems are commercially available in kit form from, inter alia, Invitrogen.

Examples of chemical synthesis technologies are solid phase synthesis and liquid phase synthesis. As a solid phase synthesis, for example, the amino acid corresponding to the carboxy-terminus of the peptide to be synthetized is bound to a support which is insoluble in organic solvents, and by alternate repetition of reactions, one wherein amino acids with their amino groups and side chain functional groups protected with appropriate protective groups are condensed one by one in order from the carboxy-terminus to the amino-terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CI-Z (2-chlorobenzyloxycarbonyl), Br-Z (2-bromobenzyloxycarbonyl), Bzl (benzyl), Fmoc (9-fluorenylmethoxycarbonyl), Mbh (4,4'-dimethoxydibenzhydryl), Mtr (4-methoxy-2,3,6-trimethylbenzenesulphonyl), Trt (trityl), Tos (tosyl), Z (benzyloxycarbonyl) and CI2-Bzl (2,6-dichlorobenzyl) for the amino groups; NO2 (nitro) and Pmc (2,2,5,7,8-pentamethylchromane-6-sulphonyl) for the guanidino groups); and tBu (t-butyl) for the hydroxyl groups). After synthesis of the desired peptide, it is subjected to the de-protection reaction and cut out from the solid support. Such peptide cutting reaction may be carried with hydrogen fluoride or tri - fluoromethane sulfonic acid for the Boc method, and with TFA for the Fmoc method. Totally synthetic chemokines are disclosed in the literature (Dawson PE et al., 1994; Brown A et al., 1996).

Purification of the natural, synthetic or recombinant antagonists of the invention can be carried out by any one of the methods known for this purpose, i.e. any conventional procedure involving extraction, precipitation, chromatography, electrophoresis, or the like. A further purification procedure that may be used in preference for purifying the protein of the invention is affinity chromatography using monoclonal antibodies or affinity groups, which bind the target protein and which are produced and immobilized on a gel matrix contained within a column. Impure preparations containing the proteins are passed through the column. The protein will be bound to the column by heparin or by the specific antibody while the impurities will pass through. After washing, the protein is eluted from the gel by a change in pH or ionic strength. Alternatively, HPLC (High Performance Liquid Chromatography) can be used. The elution can be carried-using a water-acetonitrile-based solvent commonly employed for protein purification.

Another object of the invention is represented by purified preparations of said CXCL8 antagonists. Purified preparations, as used herein, refers to the preparati ons which are at least 1 % (by dry weight), and preferably at least 5%, of said antagonists.

Another object of the present invention is the use of CXCL8 antagonists (in the form of proteins and their alternative forms described above, as well as the related peptide mimetics, cells and the nucleic acids) as medicaments, in particular as the active ingredients in the manufacture of pharmaceutical compositions for the treatment or prevention CXCL8-related diseases, such as autoimmune, inflammatory, or infectious diseases. The process for the preparation of such pharmaceutical compositions comprises combining the CXCL8 antagonist together with a pharmaceutically acceptable carrier.

The pharmaceutical compositions may contain, in combination with the CXCL8 antagonist of the invention as active ingredient, suitable pharmaceutically acceptable carriers, biologically compatible vehicles and additives which are suitable for administration to an animal (for example, physiological saline) and eventually comprising auxiliaries (like excipients, stabilizers, adjuvants, or diluents) which facilitate the processing of the active compounds into preparations which can be used pharmaceutically. The pharmaceutical compositions may be formulated in any acceptable way to meet the needs of the mode of administration. For example, the use of biomaterials and other polymers for drug delivery, as well the different techniques and models to validate a specific mode of administration, are disclosed in literature (Luo B and Prestwich GD, 2001; Cleland JL et al., 2001).

An "effective amount" refers to an amount of the active ingredients that is sufficient to affect the course and the severity of the disease, leading to the reduction or remission of such pathology. The effective amount will depend on the route of administration and the condition of the patient.

"Pharmaceutically acceptable" is meant to encompass any carrier, which does not interfere with the effectiveness of the biological activity of the active ingredient and that is not toxic to the host to which is administered. For example, for parenteral administration, the above active ingredients may be formulated in unit dosage form for injection in vehicles such as saline, dextrose solution, serum albumin and Ringer's solution. Carriers can be selected also from starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the various oils, including those of petroleum, animal, vegetable or synthetic origin (peanut oil, soybean oil, mineral oil, sesame oil).

Any accepted mode of administration can be used and determined by those skilled in the art to establ ish the desired blood levels of the active ingredients. For example, administration may be by various parenteral routes such as subcutaneous, intravenous, intradermal, intramuscular, intraperitoneal, intranasal, transdermal, oral, rectal, or buccal routes. The pharmaceutical compositions of the present invention can also be administered in sustained or controlled release dosage forms, including depot injections, osmotic pumps, and the like, for the prolonged administration of the polypeptide at a predetermined rate, preferably in unit dosage forms suitable for single administration of precise dosages.

Parenteral administration can be by bolus injection or by gradual perfusion over time. Preparations for parenteral administration include sterile aqueous or non-aqueous -solutions,--suspensions,- and emulsions, which may contain auxiliary agents or excipients known in the art, and can be prepared according to routine methods. In addition, suspension of the active compounds as appropriate oily injection suspensions may be administered. Suitable lipophilic solvents or vehicles include fatty oils, for example, sesame oil, or synthetic fatty acid esters, for example, sesame oil, or synthetic fatty acid esters, for example, ethyl oleate or triglycerides. Aqueous injecti on suspensions that may contain substances increasing the viscosity of the suspension include, for example, sodium carboxymethyl cellulose, sorbitol, and/or dextran. Optionally, the suspension may also contain stabilizers. Pharmaceutical compositions include suitable solutions for administration by injection, and contain from about 0.01 to 99.99 percent, preferably from about 20 to 75 percent of active compound together with the excipient.

It is understood that the dosage administered will be dependent upon n the age, sex, health, and weight of the recipient, kind of concurrent treatment, if any, frequency of treatment, and the nature of the effect desired. The dosage will be tailored to the individual subject, as is understood and determinable by one of skil in the art. The total dose required for each treatment may be administered by multiple doses or in a single dose. The pharmaceutical composition of the present invention may be administered alone or in conjunction with other therapeutics directed to the condition, or directed to other symptoms of the condition. Usually a daily dosage of active ingredient is comprised between 0.01 to 100 milligrams per kilogram of body weight per day. Ordinarily 1 to 40 milligrams per kilogram per day given in divided dose s or in sustained release form is effective to obtain the desired results. Second or subsequent administrations can be performed at a dosage, which is the same, less than, or greater than the initial or previous dose administered to the individual.

Also disclosed is a method for treating or preventing CXCL8-related diseases comprising the administration of an effective amount of an antagonist of CXCL8 of the present invention.

The wording "CXCL8-related diseases" indicate any disease due to an excessive or uncontrolled CXCL8 production, leading to a massive neutrophil / T-cell infiltration or neovascular growth, and wherein the administration of a CXCL8 antagonist may provide a beneficial effect. A non-exhaustive list of such chronic, acute, or inherited diseases includes: hyperproliferative diseases, auto-/immune diseases, inflammatory diseases, bacterial/fungal//protozoal/viral infections, cardiac and vascular diseases. Specific examples of such diseases are: leiomyomas, adenomas, lipomas, hemangiomas, fibromas, vascular occlusion, restenosis, atherosclerosis, cancer, neoplasia, carcinoma, psoriasis, atopic dermatitis, rheumatoid arthritis, asthma, chronic obstructive pulmonary disease, adult respiratory distress syndrome, inflammatory bowel disease, Crohn's disease, ulcerative colitis, stroke, septic shock, endotoxic shock, gram negative sepsis, toxic shock syndrome, cardiac and renal reperfusion injury, glomerulonephritis, thrombosis, graft vs. host reaction, A1zheimer's disease, allograft rejections, malaria, restinosis, angiogenesis, atherosclerosis, osteoporosis.

The therapeutic applications of the CXCL8 antagonists of the invention and of the related reagents can be evaluated (in terms or safety, pharmacokinetics and efficacy) by the means of the *in vivo* or *in vitro* assays making use of animal cell, tissues and models (Coleman R et al., 2001; Li A, 2001; Methods Mol. Biol vol. 138, "Chemokines Protocols", edited by Proudfoot A et al., Humana Press Inc., 2000; Methods Enzymol, vol. 287 and 288, Academic Press, 1997).

The present invention has been described with reference to the specific embodiments, but the content of the description comprises all modifications and substitutions, which can be brought by a person skilled in the art without extending beyond the meaning and purpose of the claims.

The invention will now be described by means of the following Examples, which should not be construed as in any way limiting the present invention. The Examples will refer to the Figures specified here below.

### EXAMPLES

### Example 1: preparation and characterization of the CXCL8 mutant sequences

### Materials and methods

### Expression of the human CXCL8 mutants.

Mature human CXCL8 and CXCL8 mutants were expressed in the yeast *Pichia pastoris* using the vector pPIC9K (Invitrogen) that allows the secretion of the cloned protein using the *S. cerevisiae* Mat α-factor pre-pro signal peptide.

The CXCL8 mutants were generated by "megaprimer" PCR mutagenesis (Sarkar G and Sommer S, 1990) of the DNA sequence coding for human CXCL8 (IL-8; NCBI Acc. N° P10145 and M23344), and in particular for the mature form, corresponding to the segment 28-99 of the precursor molecule, and containing 72 amino acids.

The mutations were confirmed by sequencing. The pPIC9K-based vectors containing the coding sequence for human CXCL8 (SEQ ID NO: 1), CXCL8-1B3 (SEQ ID NO: 3), and CXCL8-2B3 (SEQ ID NO: 5) were used to transform into *Pichia pastoris* (strain GS115-His⁻) by electroporation. His⁺ transformants were selected on minimal medium and screened for resistance to 1 mg/ml Geneticin (G418). G418-resistant clones were analyzed for secretion of the recombinant CXCL8 variants by small-scale induction with 0.5% methanol in shake flasks, and analysed by Coomassie Blue-stained SDS-PAGE.

The purification of the recombinant proteins was performed by removing the supernatant of the culture and adjusting the pH of the solution to 4.5 with acetic acid, and the conductivity to 20 mS by dilution with H₂0. The solution was applied to a HiLoad S 26/10 column previously equilibrated in 20 mM sodium acetate, (pH 4.5) and protein was eluted with a linear 0-2M NaCl gradient in the same buffer. The fractions containing the recombinant protein were pooled, dialysed against two changes of 1 % acetic acid, and finally against 0.1% trifluoroacetic acid, and then lyophilised. The authenticity of the protein was verified by mass spectrometry.

### Peritoneal cellular recruitment

Female Balb/C mice (Janvier, France) of 8 to 12 weeks were housed under normal animal holding conditions with a standard 12-hour light/dark cycle and free access to food and water.Groups composed of 3 mice were injected intraperitoneally with 200 µl of saline (sterile LPS-free NaCl 0.9% (w/v)), or of this solution containing of CXCL8 or of one its mutants at 10 µg per injection. For studies investigating the inhibitory effects of CXCL8 mutants on CXCL8-induced peritoneal cell chemotaxis, these molecules were administered intraperitoneally 30 minutes before the intraperitoneal injection of CXCL8. All the molecules were administered at the concentration and in buffer above indicated.

Peritoneal lavages to assess cell recruitment were performed at 4 hours after the chemokine, or chemokine mutant, final injection as follows. Mice were sacrificed by asphyxiation with rising concentrations of CO₂ in a plexiglass box. Skin was cleaned with 70% ethanol. The outer layer of skin was removed, exposing the peritoneal membrane. The peritoneal cavity was lavaged 3 times with 5 ml ice cold PBS and flu id was pooled in a 15 ml polystyrene Falcon tube (Becton Dickinson) on ice. Each lavage was accompanied with a light massage of the peritoneal cavity. Lavage fluid was centrifuged at 425xg, the supernatant discarded and the resultant cell pellet was resusp ended by gentle multiple pipetting in 1 ml PBS. 10 µl cell suspension was stained with 90 µl trypan blue and total cell counts were enumerated with a Neubauer haemocytometer by counting 4 areas each of 1 mm². The mean of the 4 counts was taken, multiplied by the dilution factor of 10, and multiplied again by 10 to give the number of cells per µl, according to the directions for use accompanying the haemocytometer. Finally the total value was multiplied by 1000 (to equal 1 ml) to arrive at the total cell num ber recovered.

### Results

Mature human CXCL8 was expressed in two mutated forms to identify the sequence and the properties of non-heparin binding variants. Target of the mutations were specific combinations of basic residues clustered in the C-terminus of mature human CXCL8 (SEQ ID NO: 2), either Arginine60-Lysine64-Lysine67 (CXCL8-1B3; SEQ ID NO: 4) or Lysine64-Lysine67-Arginine68 (CXCL8-2B3; SEQ ID NO: 6), that have been mutated to Alanine. The residues located in this region are known to be involved in heparin binding but, apart from being spatially distinct from those involved in receptor binding, the effect of their mutation was studied at the level of single residue and not in connection to any specific CXCL8 antagonistic activity (Frevert C et al., 2003; Kuschert G et al., 1998)

The ability of the mutants to modulate *in vivo* chemotaxis was tested in the peritoneal cell recruitment model to examine whether the disruption of the GAG binding site of CXCL8 may lead to molecules affecting CXCL8-induced cell recruitment *in vivo.*

Neither CXCL8 mutants induced a significant increase over baseline compared with the increase in cells recovered from mice treated with the parent chemokine (Fig. 2A), but both molecules are capable to antagonize CXCL8-mediated recruitment of cells in the peritoneal cell chemotaxis model with a high degree of significance (Fig. 2B).

In conclusion, CXCL8-1B3 and CXCL8-2B3 shows no recruitment proprieties of its own, but considerable antagonistic activities over CXCL8, with respect of cellular recruitment induction into the peritoneum, when prior administered at the same dose (10 µg/mouse). Therefore, the combined substitution of specific basic residues in the C-terminal region of CXCL8 produces CXCL8 antagonists capable of inhibiting *in vivo* the cellular recruitment induced by CXCL8.

**TABLE I**

| AminoAcid | Synonymous Group | More Preferred Synonymous Groups |
|---|---|---|
| **Ser** | Gly, Ala, Ser, Thr, Pro | Thr, Ser |
| **Arg** | Asn, Lys, Gln, Arg, His | Arg, Lys, His |
| **Leu** | Phe, Ile, Val, Leu, Met | Ile, Val, Leu, Met |
| **Pro** | Gly, Ala, Ser, Thr, Pro | Pro |
| **Thr** | Gly, Ala, Ser, Thr, Pro | Thr, Ser |
| **Ala** | Gly, Thr, Pro, Ala, Ser | Gly, Ala |
| **Val** | Met, Phe, Ile, Leu, Val | Met, Ile, Val, Leu |
| **Gly** | Ala, Thr, Pro, Ser, Gly | Gly, Ala |
| **Ile** | Phe, Ile, Val, Leu, Met | Ile, Val, Leu, Met |
| **Phe** | Trp, Phe,Tyr | Tyr, Phe |
| **Tyr** | Trp, Phe,Tyr | Phe, Tyr |
| **Cys** | Ser, Thr, Cys | Cys |
| **His** | Asn, Lys, Gln, Arg, His | Arg, Lys, His |
| **Gln** | Glu, Asn, Asp, Gln | Asn, Gln |
| **Asn** | Glu, Asn, Asp, Gln | Asn, Gln |
| **Lys** | Asn, Lys, Gln, Arg, His | Arg, Lys, His |
| **Asp** | Glu, Asn, Asp, Gln | Asp, Glu |
| **Glu** | Glu, Asn, Asp, Gln | Asp, Glu |
| **Met** | Phe, Ile, Val, Leu, Met | Ile, Val, Leu, Met |
| **Trp** | Trp, Phe,Tyr | Trp |

**TABLE II**

| **Amino Acid** | **Synonymous Group** |
|---|---|
| Ser | D-Ser, Thr, D-Thr, allo-Thr, Met, D-Met, Met(O), D-Met(O), L-Cys, D-Cys |
| Arg | D-Arg, Lys, D-Lys, homo-Arg, D-homo-Arg, Met, Ile, D-.Met, D-Ile, Orn, D-Om |
| Leu | D-Leu, Val, D-Val, AdaA, AdaG, Leu, D-Leu, Met, D-Met |
| Pro | D-Pro, L-I-thioazolidine-4-carboxylic acid, D-or L-1-oxazolidine-4-carboxylic acid |
| Thr | D-Thr, Ser, D-Ser, allo-Thr, Met,D-Met, Met(O), D-Met(O), Val, D-Val |
| Ala | D-Ala, Gly, Aib, B-Ala, Acp, L-Cys, D-Cys |
| Val | D-Val, Leu, D-Leu, Ile, D-Ile, Met, D-Met, AdaA, AdaG |
| Gly | Ala, D-Ala, Pro, D-Pro, Aib, .beta.-Ala, Acp |
| Ile | D-Ile, Val, D-Val, AdaA, AdaG, Leu, D-Leu, Met, D-Met |
| Phe | D-Phe, Tyr, D-Thr, L-Dopa, His, D-His, Trp, D-Trp, Trans-3,4, or 5-phenylproline, AdaA, AdaG, cis-3,4, or 5-phenylproline, Bpa, D-Bpa |
| Tyr | D-Tyr, Phe, D-Phe, L-Dopa, His, D-His |
| Cys | D-Cys, S-Me-Cys, Met, D-Met, Thr, D-Thr |
| Gln | D-Gln, Asn, D-Asn, Glu, D-Glu, Asp, D-Asp |
| Asn | D-Asn, Asp, D-Asp, Glu, D-Glu, Gln, D-Gln |
| Lys | D-Lys, Arg, D-Arg, homo-Arg, D-homo-Arg, Met, D-Met, Ile, D-Ile, Om, D-Om |
| Asp | D-Asp, D-Asn, Asn, Glu, D-Glu, Gln, D-Gln |
| Glu | D-Glu, D-Asp, Asp, Asn, D-Asn, Gln, D-Gln |
| Met | D-Met, S-Me--Cys, Ile, D-Ile, Leu, D-Leu, Val, D-Val |

### REFERENCES

Ali S et al., Biochem J , 358: 737-745, 2001.
Atta-ur-Rahman H and Siddiqui RA, Curr Pharm Des, 5: 241-253, 1999.
Baggiolini M et al., Annu Rev Immunol, 15: 675-705, 1997.
Baggiolini M, J Intern Med, 250: 91-104, 2001.
Brown A et al., J Pept Sci, 2:40-46, 1996.
Cleland JL et al., Curr Opin Biotechnol, 12: 212-9, 2001.
Coleman R et al., Drug Discov Today, 6: 1116-1126, 2001.
Dawson PE et al., Science, 266: 776-9, 1994.
Dias-Baruffi M et al., Glycoconj J, 15: 523-526, 1998.
Dougherty DA, Curr Opin Chem Biol, 4: 645-52, 2000.
Fernandez EJ and Lolis E, Annu Rev Pharmacol Toxicol, 42:469-499, 2002.
Frevert C et al., J Immunol, 168: 3550-3556, 2002.
Frevert C et al., Am J Respir Cell Mol Biol, 28: 464-472, 2003.
Godessart N and Kunkel SL, Curr Opin Immunol, 13: 670-675, 2001.
Goger B et al., Biochemistry, 41: 1640-1646, 2002.
Golebiowski A et al., Curr Opin Drug Discov Devel, 4: 428-34, 2001.
Greenwald RB et al., Adv Drug Deliv Rev, 55: 217-250, 2003.
Harris JM and Chess RB, Nat Rev Drug Discov, 2: 214-221, 2003.
Hoogewerf AJ et al., Biochemistry, 36: 13570-13578, 1997.
Hruby VJ and Balse PM, Curr Med Chem, 7: 945-970, 2000.
Kuschert G et al., Methods Enzymol, 287:369-378, 1997.
Kuschert G et al., Biochemistry, 37: 11193-11201, 1998.
Kuschert G et al., Biochemistry, 38: 12959-12968, 1999.
Li A, Drug Discov Today, 6: 357-366, 2001.
Loetscher P and Clark-Lewis I, J Leukoc Biol, 69: 881-884, 2001.
Lortat-Jacob H et al., Proc Natl Acad Sci U S A, 99: 1229-1234, 2002.
Luo B and Prestwich GD, Exp Opin T her Patents, 11: 1395-1410, 2001.
Moser B et al., J Biol Chem, 268: 7125-7128, 1993.
Mukaida N, Am J Physiol Lung Cell Mol Physiol, 284: L566-577, 2003.
Murphy LR et al., Protein Eng, 13:149-152, 2000.
Nilsson J et al., Protein Expr Purif, 11: 1-16, 1997.
Patel D et al., Clin Immunol, 99: 43-52, 2001.
Pillai O and Panchagnula R, Cur Opin Chem Biol, 5: 447-451, 2001
Proudfoot A, et al. J Biol Chem 276: 10620-10626, 2001.
Proudfoot A et al., Proc Natl Acad Sci U S A, 100: 1885-1890, 2003.
Rogov SI and Nekrasov AN, Protein Eng, 14: 459-063, 2001.
Sarkar G and Sommer S, Biotechniques, 8: 404-407, 1990.
Schwarz MK and Wells TN, Curr Opin Chem Biol, 3: 407-417, 1999,
Shi Q et al., J Interferon Cytokine Res, 21: 553-566, 2001.
Skelton N et al., Structure Fold Des, 7: 157-68, 1999.
Spillmann D et al., J Biol Chem, 273: 15487-15493, 1998.
Villain M et al., Chem Biol, 8: 673-679, 2001.
Webb L et al., Proc Natl Acad Sci U S A, 90: 7158-7162, 1993.
Witt D and Lander A, Curr Biol, 4: 394-400, 1994.
Zeilhofer HU and Schorr W, Curr Opin Hematol, 7: 178-182, 2000.

## Claims

1. A CXCL8 antagonist comprising a mutant sequence of human mature CXCL8 polypeptide identified by SEQ ID NO: 2, **characterized in that** the three basic residues Arginine 60, Lysine 64 and Lysine 67 of said polypeptide are substituted to Alanine, Glycine, Serine, Threonine, Proline, Glutamic Acid, Glutamine, Aspartic Acid, or Asparagine.

2. The CXCL8 antagonist of claim 1 **characterized in that** the mutant sequence is CXCL8-1B3 identified by SEQ ID NO: 4.

3. The CXCL8 antagonist of claim 1 or 2, wherein one or more amino acids have been added, deleted, or substituted belonging to the first six amino acids in the amino-terminal domain of the mature human CXCL8.

4. The CXCL8 antagonist of any of the claims from 1 to 3, **characterized in that** said antagonist comprises an amino acid sequence belonging to a protein sequence other than human mature CXCL8.

5. The antagonist of claim 4, **characterized in that** said antagonist comprises the amino acid sequence belonging to one or more of these protein sequences: extracellular domains of membrane-bound protein, immunoglobulin constant region (Fc region), multimerization domains, signal peptides, export signal-containing proteins, and tag sequences.

6. The antagonist of any of the claims from 1 to 5, **characterized in that** a conjugate or complex is formed with a molecule chosen amongst radioactive labels, biotin, fluorescent labels, cytotoxic agents, or drug delivery agents.

7. The nucleic acid molecule comprising the DNA sequence coding for a CXCL8 antagonist of any of the claims from 1 to 5.

8. The expression vector comprising the DNA molecule of claim 7.

9. A host cell transformed with a vector of claim 8.

10. Process for the preparation of a CXCL8 antagonist of any of the claims from 1 to 6, comprising culturing the transformed cells of claim 9 and collecting the expressed proteins.

11. A purified preparation containing at least 1 % of the CXCL8 antagonist of claims from 1 to 6, or of the nucleic acid of claim 7.

12. A CXCL8 antagonist of any of the claims from 1 to 6, a DNA of claim 7, a vector of claim 8 or a cell of claim 9, for use as a medicament.

13. A pharmaceutical composition containing a CXCL8 antagonist of any of the claims from 1 to 6 as active ingredient.

14. The use of a CXCL8 antagonist of any of the claims from 1 to 6 for the manufacture of a medicament for the treatment of autoimmune, inflammatory or infectious diseases.

15. A CXCL8 antagonist of any of the claims from 1 to 6 for the treatment of autoimmune, inflammatory or infectious diseases.

## Patentansprüche

1. CXCL8-Antagonist, umfassend eine mutierte Sequenz von menschlichem reifem, durch SEQ ID NO: 2 identifiziertem CXCL8-Polypeptid, **dadurch gekennzeichnet, dass** die drei basischen Reste Arginin 60, Lysin 64 und Lysin 67 des Polypeptids durch Alanin, Glycin, Serin, Threonin, Prolin, Glutaminsäure, Glutamin, Asparaginsäure oder Asparagin substituiert sind.

2. CXCL8-Antagonist nach Anspruch 1, **dadurch gekennzeichnet, dass** es sich bei der mutierten Sequenz um durch SEQ ID NO: 4 identifiziertes CXCL8-1 B3 handelt.

3. CXCL8-Antagonist nach Anspruch 1 oder 2, wobei eine oder mehrere zu den ersten sechs Aminosäuren in der aminoterminalen Domäne des reifen menschlichen CXCL8 gehörende Aminosäuren addiert, deletiert oder substituiert worden sind.

4. CXCL8-Antagonist nach einem der Ansprüche 1 bis 3, **dadurch gekennzeichnet, dass** der Antagonist eine Aminosäuresequenz umfasst, die zu einer anderen Proteinsequenz als der des menschlichen reifen CXCL8 gehört.

5. Antagonist nach Anspruch 4, **dadurch gekennzeichnet, dass** der Antagonist die Aminosäuresequenz umfasst, die zu einer oder mehrerer dieser Proteinsequenzen gehört: extrazelluläre Domänen von membrangebundenem Protein, eine konstante Immunglobulinregion (Fc-Region), Multimerisierungsdomänen, Signalpeptide, Proteine, die Exportsignale enthalten, und Anhang-Sequenzen.

6. Antagonist nach einem der Ansprüche 1 bis 5, **dadurch gekennzeichnet, dass** ein Konjugat oder Komplex mit einem Molekül gebildet wird, das aus radioaktiven Markierungen, Biotin, Fluoreszenzmarkierungen, zytotoxischen Mitteln oder Mitteln zur Arzneistoffabgabe ausgewählt ist.

7. Nucleinsäuremolekül, umfassend die DNA-Sequenz, die einen CXCL8-Antagonisten nach einem der Ansprüche 1 bis 5 kodiert.

8. Expressionsvektor, umfassend das DNA-Molekül nach Anspruch 7.

9. Wirtszelle, transformiert mit einem Vektor nach Anspruch 8.

10. Verfahren zur Herstellung eines CXCL8-Antagonisten nach einem der Ansprüche 1 bis 6, umfassend Züchten der transformierten Zellen nach Anspruch 9 und Gewinnen der exprimierten Proteine.

11. Gereinigte Zubereitung, die mindestens 1 % des CXCL8-Antagonisten nach den Ansprüchen 1 bis 6 oder der Nucleinsäure nach Anspruch 7 enthält.

12. CXCL8-Antagonist nach einem der Ansprüche 1 bis 6, DNA nach Anspruch 7, Vektor nach Anspruch 8 oder Zelle nach Anspruch 9 zur Verwendung als Medikament.

13. Pharmazeutische Zusammensetzung, die einen CXCL8-Antagonisten nach einem der Ansprüche 1 bis 6 als wirksamen Bestandteil enthält.

14. Verwendung eines CXCL8-Antagonisten nach einem der Ansprüche 1 bis 6 zur Herstellung eines Medikaments zur Behandlung von Autoimmun-, entzündlichen oder infektiösen Erkrankungen.

15. CXCL8-Antagonist nach einem der Ansprüche 1 bis 6 zur Behandlung von Autoimmun-, entzündlichen oder infektiösen Erkrankungen.

## Revendications

1. Antagoniste de CXCL8 comprenant une séquence mutante d'un polypeptide humain mature CXCL8 identifié par la séquence SEQ ID NO :2, **caractérisé en ce que** les trois résidus basiques Arginine 60, Lysine 64 et Lysine 67 dudit polypeptide sont substitués par Alanine, Glycine, Sérine, Thréonine, Proline, Acide glutamique, Glutamine, Acide aspartique ou Asparagine.

2. Antagoniste de CXCL8 selon la revendication 1, **caractérisé en ce que** la séquence mutante est CXCL8-1B3 identifiée par SEQ ID NO :4.

3. Antagoniste de CXCL8 selon la revendication 1 ou 2, dans lequel un ou plusieurs acides aminés ont été ajoutés, délétés ou substitués parmi ceux appartenant aux six premiers acides aminés du domaine amino-terminal de CXCL8 humain mature.

4. Antagoniste de CXCL8 selon l'une quelconques des revendications 1 à 3, **caractérisé en ce que** ledit antagoniste comprend une séquence en acides aminés appartenant à un séquence protéique autre que celle du CXCL8 mature humain.

5. Antagoniste selon la revendication 4, **caractérisé en ce que** ledit antagoniste comprend la séquence en acides aminés appartenant à une ou plusieurs des séquences protéiques suivantes : les domaines extracellulaires d'une protéine liée à la membrane, une région constante de l'immunoglobuline (région Fc), les domaines de multimérisation, des peptides-signal, des protéines contenant un signal d'export, et des séquences étiquettes.

6. Antagoniste selon l'une quelconque des revendications 1 à 5, **caractérisé en ce qu'**un conjugué ou un complexe est formé avec une molécule choisie parmi des marqueurs radioactifs, la biotine, des marqueurs fluorescents, des agents cytotoxiques ou des agents délivrant des drogues.

7. Molécule d'acide nucléique comprenant la séquence d'ADN codant pour un antagoniste de CXCL8 selon l'une quelconque des revendications 1 à 5.

8. Vecteur d'expression comprenant la molécule d'ADN selon la revendication 7.

9. Cellule-hôte transformée par un vecteur selon la revendication 8.

10. Procédé pour la préparation d'un antagoniste de CXCL8 selon l'une quelconque des revendications 1 à 6, comprenant le fait de mettre en culture les cellules transformées de la revendication 9, et le fait de récolter les protéines exprimées.

11. Préparation purifiée contenant au moins 1% d'antagoniste de CXCL8 selon les revendications 1 à 6, ou l'acide nucléique de la revendication 7.

12. Antagoniste de CXCL8 selon l'une quelconque des revendications 1 à 6, ou ADN de la revendication 7, ou vecteur de la revendication 8 ou cellule selon la revendication 9, pour son utilisation comme médicament.

13. Composition pharmaceutique contenant un antagoniste de CXCL8 selon l'une quelconque des revendications 1 à 6 en tant qu'ingrédient actif.

14. Utilisation d'un antagoniste de CXCL8 selon l'une quelconque des revendications 1 à 6, pour la préparation d'un médicament pour le traitement des maladies auto-immunes, inflammatoires ou infectieuses.

15. Antagoniste de CXCL8 selon l'une quelconque des revendications 1 à 6, pour le traitement des maladies auto-immunes, inflammatoires ou infectieuses.


### SEQUENCE LISTING

<110> Applied Research Systems ARS Holding N.V.
<120> NOVEL CXCL8 ANTAGONISTS
<130> WO932
<160> 6
<170> PatentIn version 3.1
<210> 1
   <211> 297
   <212> DNA
   <213> homo sapiens
   <220>
   <223> Human CXCL8 coding sequence
<400> 1
   atgacttcca agctggccgt ggctctcttg gcagccttcc tgatttctgc agctctgtgt     60
   gaaggtgcag ttttgccaag gagtgctaaa gaacttagat gtcagtgcat aaagacatac    120
   tccaaacctt tccaccccaa atttatcaaa gaactgagag tgattgagag tggaccacac    180
   tgcgccaaca cagaaattat tgtaaagctt tctgatggaa gagagctctg tctggacccc    240
   aaggaaaact gggtgcagag ggttgtggag aagtttttga agagggctga gaattca       297
<210> 2
   <211> 72
   <212> PRT
   <213> Homo sapiens
   <220>
   <223> Mature human CXCL8
<400> 2
   Ser Ala Lys Glu Leu Arg Cys Gln Cys Ile Lys Thr Tyr Ser Lys Pro
   1               5                   10                  15
   Phe His Pro Lys Phe Ile Lys Glu Leu Arg Val Ile Glu Ser Gly Pro
               20                  25                  30
   His Cys Ala Asn Thr Glu Ile Ile Val Lys Leu Ser Asp Gly Arg Glu
           35                  40                  45
   Leu Cys Leu Asp Pro Lys Glu Asn Trp Val Gln Arg Val Val Glu Lys
       50                  55                  60
   Phe Leu Lys Arg Ala Glu Asn Ser
   65                  70
<210> 3
   <211> 297
   <212> DNA
   <213> Synthetic construct
   <220>
   <223> CXCL8-1B3 coding sequence
<400> 3
   atgacttcca agctggccgt ggctctcttg gcagccttcc tgatttctgc agctctgtgt    60
   gaaggtgcag ttttgccaag gagtgctaaa gaacttagat gtcagtgcat aaagacatac   120
   tccaaacctt tccaccccaa atttatcaaa gaactgagag tgattgagag tggaccacac   180
   tgcgccaaca cagaaattat tgtaaagctt tctgatggaa gagagctctg tctggacccc   240
   aaggaaaact gggtgcaggc ggttgtggag gcgtttttgg cgagggctga gaattca      297
<210> 4
   <211> 72
   <212> PRT
   <213> Synthetic construct
   <220>
   <223> Mature CXCL8-1B3
<400> 4
   Ser Ala Lys Glu Leu Arg Cys Gln Cys Ile Lys Thr Tyr Ser Lys Pro
   1               5                   10                  15
   Phe His Pro Lys Phe Ile Lys Glu Leu Arg Val Ile Glu Ser Gly Pro
               20                  25                  30
   His Cys Ala Asn Thr Glu Ile Ile Val Lys Leu Ser Asp Gly Arg Glu
           35                  40                  45
   Leu Cys Leu Asp Pro Lys Glu Asn Trp Val Gln Ala Val Val Glu Ala
       50                  55                  60
   Phe Leu Ala Arg Ala Glu Asn Ser
   65                  70
<210> 5
   <211> 297
   <212> DNA
   <213> Synthetic construct
   <220>
   <223> CXCL8-2B3 coding sequence
<400> 5
   atgacttcca agctggccgt ggctctcttg gcagccttcc tgatttctgc agctctgtgt     60
   gaaggtgcag ttttgccaag gagtgctaaa gaacttagat gtcagtgcat aaagacatac    120
   tccaaacctt tccaccccaa atttatcaaa gaactgagag tgattgagag tggaccacac    180
   tgcgccaaca cagaaattat tgtaaagctt tctgatggaa gagagctctg tctggacccc    240
   aaggaaaact gggtgcagag ggttgtggag gcgtttttgg cggcggctga gaattca       297
<210> 6
   <211> 72
   <212> PRT
   <213> Synthetic construct
   <220>
   <223> Mature CXCL8-2B3
<400> 6
   Ser Ala Lys Glu Leu Arg Cys Gln Cys Ile Lys Thr Tyr Ser Lys Pro
   1               5                   10                  15
   Phe His Pro Lys Phe Ile Lys Glu Leu Arg Val Ile Glu Ser Gly Pro
               20                  25                  30
   His Cys Ala Asn Thr Glu Ile Ile Val Lys Leu Ser Asp Gly Arg Glu
           35                  40                  45
   Leu Cys Leu Asp Pro Lys Glu Asn Trp Val Gln Arg Val Val Glu Ala
       50                  55                  60
   Phe Leu Ala Ala Ala Glu Asn Ser
   65                  70